# EUROPEAN PATENT SPECIFICATION

(11) **EP 3 302 326 B1**
(45) Date of publication and mention of the grant of the patent: **24.10.2018**
(21) Application number: 16725794.8
(22) Date of filing: 18.05.2016
(51) Int. Cl.: A61B 18/04, H05H 1/24, A61B 18/14, A61B 18/00

(54) **A DEVICE FOR TREATING SKIN USING NON-THERMAL PLASMA**
VORRICHTUNG ZUR BEHANDLUNG VON HAUT MIT NICHTTHERMISCHEM PLASMA
DISPOSITIF DE TRAITEMENT DE LA PEAU AU MOYEN D'UN PLASMA NON THERMIQUE

(30) Priority: 29.05.2015 EP 15169878
(43) Date of publication of application: 11.04.2018
(73) Proprietor: Koninklijke Philips N.V., 5656 AE Eindhoven (NL)
(72) Inventor: NIJDAM, Jeroen Christian, 5656 AE Eindhoven (NL); ZUIDERVAART, Jasper, 5656 AE Eindhoven (NL); VAN DER ZWAN, Eduard Antonius, 5656 AE Eindhoven (NL); MOOIBROEK, Stephan, 5656 AE Eindhoven (NL)
(74) Representative: van Oudheusden-Perset, Laure E.
(86) International application number: PCT/EP2016/061055
(87) International publication number: WO 2016/192986

(56) References cited:
- WO-A1-2008/131407
- WO-A1-2014/023276
- KR-B1- 101 262 632
- US-A1- 2011 306 006
- US-A1- 2014 180 276
- US-A1- 2014 222 105
- US-A1- 2015 005 681
- US-A1- 2015 112 300

## Description

### FIELD OF THE INVENTION

The present invention relates to a device for treating skin using non-thermal plasma. The present invention also relates to an electrode head assembly for use in a device for treating skin using non-thermal plasma.

### BACKGROUND OF THE INVENTION

Plasma is usually defined as an overall electrically neutral gas containing unbound positive and negative particles, such as ions and electrons, and is in particular well-known for its sterilising properties. When such a gas exists at a very high temperature in a stable state, i.e. a state in which ions and electrons are in thermal equilibrium with each other, it is called thermal plasma (or "hot plasma"). Non-thermal plasma (also known as "cold plasma", "low-temperature plasma" or "non-equilibrium plasma") may also exist, in which ions are at a much lower temperature than free electrons, e.g. human body temperature. Non-thermal plasma is therefore suitable for use in many applications such as removal of contaminants from a human body surface, without causing significant thermal tissue damage.

Devices for treating skin using non-thermal plasma are known. Such devices usually comprise a non-thermal plasma source comprising a pair of electrodes and a high voltage power supply. One of the electrodes is located at a frond end of the device. To generate non-thermal plasma, a high voltage is applied between the electrodes by the high voltage power supply, thereby creating electrical discharges between the electrodes. Such electrical discharges ionize air located between the electrodes, thereby generating non-thermal plasma. To treat a region of a human body, for example to disinfect a wound, the front end of the device is placed in contact with or close to the wound. Then, the non-thermal plasma source is activated to generate non-thermal plasma onto the wound and thereby disinfecting the wound. Such a device is disclosed in US 2011/0306006 A1.

Other devices for plasma treatment of skin are disclosed by WO2014/023276, US2014/0180276 and US2015112300. One problem is that such devices require high voltage electrical components to produce a high voltage between the electrodes in order to generate non-thermal plasma. Production of such high voltages usually requires expensive electrical components. In addition, these electrical components must be spaced by big air gaps and creepage distances, which leads to bulky electrical circuits. Furthermore, the use of such high voltages in a personal care device may be unsafe for the user.

### SUMMARY OF THE INVENTION

The invention is defined by the device of independent claim 1 and by the electrode head assembly of independent claim 9. Preferred embodiments are defined by the dependent claims. Any example or embodiment which does not fall under the scope of the claims is not part of the invention. It is an object of the invention to provide a device for treating skin using non-thermal plasma which substantially alleviates or overcomes the problems mentioned above. In particular, at least in certain embodiments, the present invention sets out to provide a device for treating skin using non-thermal plasma which is compact, cheap and safe for the user.

According to the present invention, there is provided a device for treating skin using non-thermal plasma comprising:
an electrode head assembly having a skin interface electrode for application to skin during treatment, a transformer configured to change a low voltage electrical signal into a higher voltage electrical signal, and a generator to receive said higher voltage electrical signal and generate non-thermal plasma at said skin interface electrode, and
a handle portion containing a driving device comprising a power source to generate said low voltage electrical signal,
wherein the electrode head assembly and the driving device include cooperating elements to releasably mount the electrode head assembly to the driving device and electrically connect the power source to the transformer. The device is therefore configured such that no high voltage electrical signal is transmitted through the cooperating elements. A user can detach the electrode head assembly from the driving device without any risks. The device is therefore safe for the user. Moreover, as the higher voltage electrical signal is generated by means of the transformer in the electrode head assembly, no high voltage electrical components are needed in the driving device. As such high voltages electrical components are usually expensive, this allows a low-cost manufacturing of the device. In addition, as such high voltage electrical components usually lead to bulky electrical circuits, this ensures that the device remains compact.

The cooperating elements may comprise a plug and a socket, the plug being configured to releasably engage with the socket to releasably mount the electrode head assembly to the driving device and electrically connect the power source to the transformer. This provides the advantage that the electrode head assembly is easily detachable from the remainder of the device, for example if the user wants to change of electrode head assembly or wash the electrode head assembly.

The plug may be part of the electrode head assembly and the socket may be part of the driving device.

The cooperating elements may comprise a pair of plugs and a pair of sockets, each plug being configured to respectively releasably engage with a socket to releasably mount the electrode head assembly to the driving device and electrically connect the power source to the transformer.

The electrode head assembly may comprise a body and the transformer may be embedded within an insulating material in the body. The insulating material provides electrical insulation from high electrical voltages generated within the electrode head assembly during use, for example in the transformer, thereby enabling the electrode head assembly to be safe for the user. This also provides the advantage that the electrode head assembly can be washed without any risk.

The insulating material may be hardenable and suitable for curing around the transformer. The insulating material may be a thermosetting polymer. This provides the advantage that the electrode head assembly remains compact.

The generator may comprise a main electrode and a dielectric material disposed between the main electrode and the skin interface electrode. The main electrode may be connected to the transformer so that the higher voltage electrical signal is applied to the main electrode. The higher voltage electrical signal is applied between the main electrode and the skin interface electrode. The skin interface electrode remains at a low or zero electrical potential. This ensures that, should a user accidentally touch the skin interface electrode, little or no current will pass avoiding any injury.

According to a further aspect of the invention, there is provided an electrode head assembly mountable to a driving device comprising a power source, the electrode head assembly comprising a skin interface electrode, a transformer configured to change a low voltage electrical signal received from the power source into a higher voltage electrical signal, and a generator to receive said higher voltage electrical signal and generate non-thermal plasma at said skin interface electrode,
wherein the electrode head assembly includes a cooperating element to releasably mount the electrode head assembly to the driving device and to electrically connect the power source to the transformer.

The cooperating element may comprise a plug configured to releasably engage with a socket in the driving device to releasably mount the electrode head assembly to the driving device and electrically connect the power source to the transformer.

The cooperating element may comprise a pair of plugs, each plug being configured to respectively releasably engage with a socket in the driving device to releasably mount the electrode head assembly to the driving device and electrically connect the power source to the transformer.

The electrode head assembly may comprise a body and the transformer may be located in the body and embedded within an insulating material in the body.

The insulating material may be hardenable and suitable for curing around the transformer. The insulating material may be a thermosetting polymer.

The generator may comprise a main electrode and a dielectric material disposed between the main electrode and the skin interface electrode. The main electrode may be connected to the transformer so that the higher voltage electrical signal is applied to the main electrode.

These and other aspects of the invention will be apparent from and elucidated with reference to the embodiments described hereinafter.

### BRIEF DESCRIPTION OF THE DRAWINGS

Embodiments of the invention will now be described, by way of example only, with reference to the accompanying drawings, in which:
Figure 1 shows an exploded view of a device for treating skin using non-thermal plasma which may include embodiments of the present invention;
Figure 2 shows a perspective view of the skin treating device of Figure 1;
Figure 3 shows a diagrammatic cross-sectional view of a skin treating device which may include embodiments of the present invention;
Figure 4 shows a diagrammatic circuit diagram of the skin treating device of Figure 3;
Figure 5A shows a top view of the skin treating device of Figure 1;
Figure 5B shows a cross-sectional view of the skin treating device of Figure 1 taken along the line A-A shown in Figure 5A;
Figure 5C shows an enlarged view of a detail of the skin treating device of Figure 5B;
Figure 5D shows a cross-sectional view of the skin treating device of Figure 1 taken along the line B-B shown in Figure 5A;
Figure 5E shows an enlarged view of a detail of the skin treating device of Figure 5D;
Figure 6 shows a partial diagrammatic cross-sectional view of the electrode head assembly of the skin treating device of Figure 1;
Figure 7A shows a diagrammatic perspective view of a device for treating skin using non-thermal plasma which may include embodiments of the present invention; and
Figure 7B shows a diagrammatic cross-sectional view of the skin treating device of Figure 7A.

### DETAILED DESCRIPTION OF THE EMBODIMENTS

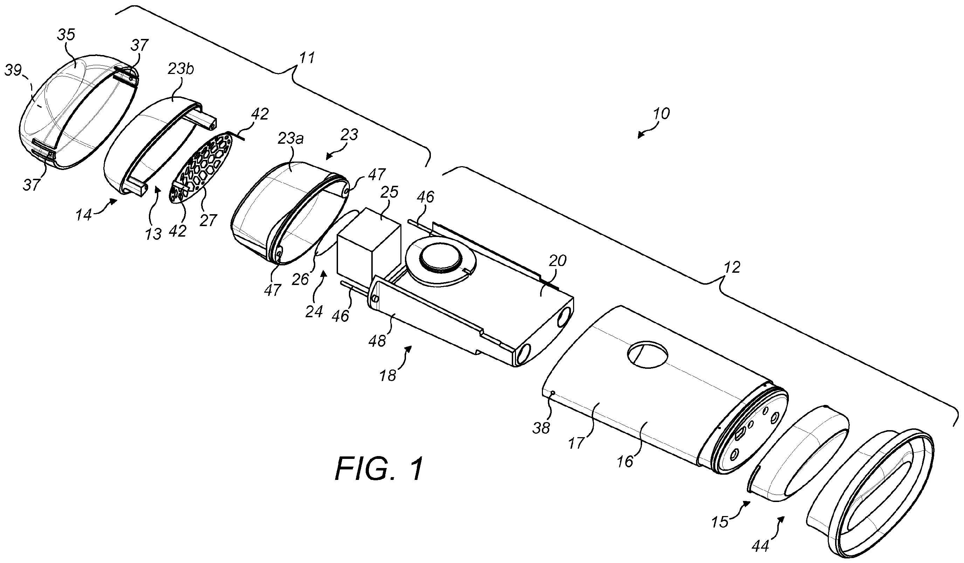

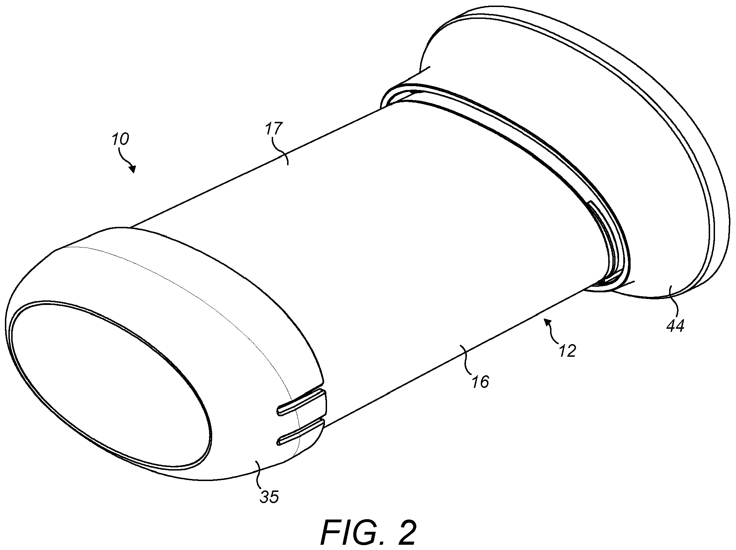

Referring to Figures 1 to 6, a skin treating device 10 according to a first embodiment of the present invention is shown. The device 10 is configured to treat skin using non-thermal plasma.

In the context of this application, the terms "non-thermal plasma", "cold plasma", "low-temperature plasma" or "non-equilibrium plasma" are equivalent. Non-thermal plasma has a temperature of less than about 40° C, i.e. a temperature tolerable to a person or user without causing injury or discomfort. In the context of this application, the terms "sterilise", "disinfect" and "decontaminate" mean that at least some of the microorganisms present on the surface of the skin are killed and/or rendered non-infectious. The terms "distal" and "proximal" herein respectively refer to as relatively closer to the skin to be treated and relatively further away from the skin to be treated.

In the present arrangement, the device 10 is configured to disinfect, sterilise or decontaminate a human or animal body surface, for example a part of the body in which bacteria are to be removed, such as an armpit, or a wound. The device 10 is configured to be hand-held. Therefore, the device 10 is of a mass, size and shape enabling a user to operate the device 10 for treating skin.

As shown in Figure 1, the device 10 comprises an electrode head assembly 11 and a handle portion 12. The device 10 comprises a distal end 14 and a proximal end 15. The electrode head assembly 11 has a skin interface 13 located at the distal end 14 and suitable for application to skin during treatment.

The handle portion 12 comprises a housing 16 having a sidewall 17. The housing 16 accommodates the electrode head assembly 11 and a driving device 18 for driving the electrode head assembly 11. As will be explained in more detail below, the electrode head assembly 11 is releasably mounted to the driving device 18. It should be noted that Figure 1, which illustrates an arrangement in which the electrode head assembly 11 is fixedly mounted to the driving device 18, is described herein for purposes of clarity only.

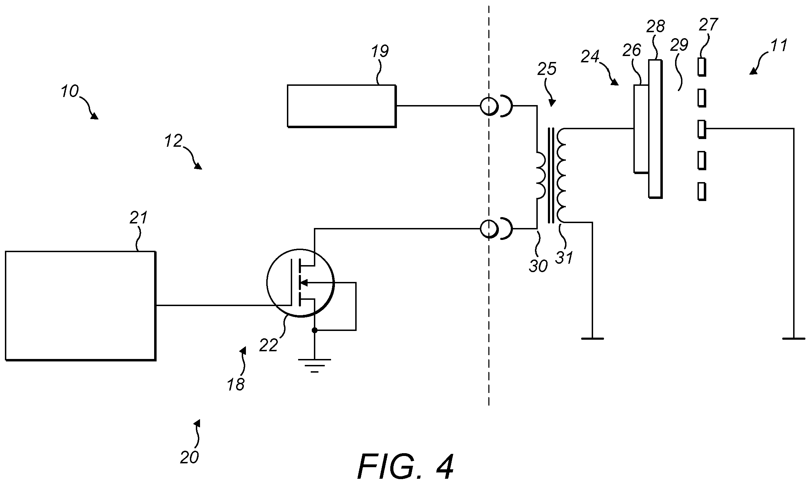

As shown in Figure 4, the driving device 18 comprises a DC voltage source 19, such as a battery, and an electrical circuit 20. In the present arrangement, the electrical circuit 20 comprises a transistor-transistor logic (TTL) circuit 21 combined with a metal oxide semiconductor field effect transistor (MOSFET) 22. The output of the TTL circuit 21 is connected to the gate of the MOSFET 22. The driving device 18 is configured to generate a low voltage electrical signal. The voltage of the low voltage electrical signal generated by the driving device 18 ranges between about 15V and about 30V, and is in particular approximately equal to 24V. A switch (not shown) is provided on the sidewall 17 of the housing 16 to enable the user to switch on or switch off the driving device 18.

The electrode head assembly 11 comprises a body 23 having a first body portion 23a and a second body portion 23b and which accommodates a generator 24 for generating non-thermal plasma and a transformer 25 to supply a high voltage electrical signal to the generator 24.

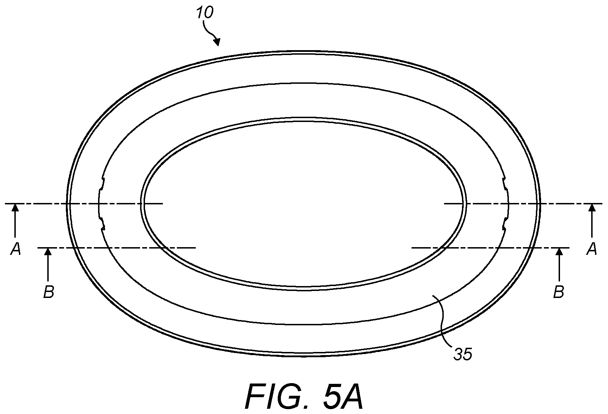

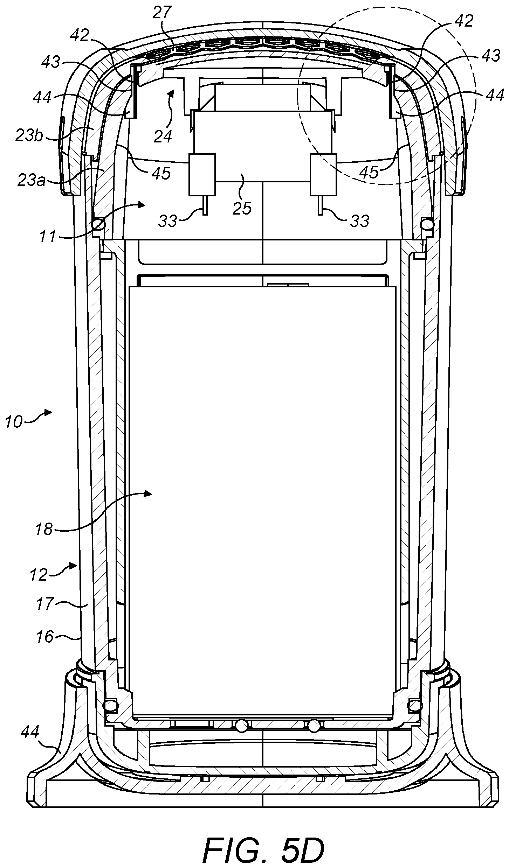

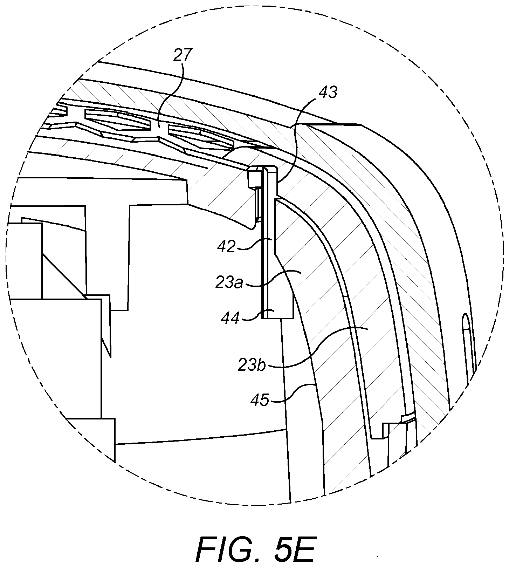

The generator 24 comprises a main electrode 26 and a skin interface electrode 27, or counter-electrode. The main electrode 26 is located in the first body portion 23a. The main electrode 26 is in the form of a plate of conductive material. The skin interface electrode 27 is located at the skin interface 13 of the device 10 and is suitable for application to skin during treatment. The skin interface electrode 27 is mounted to the body 23. As visible in Figures 5D and 5E, the skin interface electrode 27 comprises a pair of lugs 42 which are inserted in corresponding slots 43 formed in the first body portion 23a. The lugs 42 tightly fit in the slots 43 such that the skin interface electrode 27 is securely mounted to the first body portion 23a. Each lug 42 comprises a protruding element 44 abutting against an inner surface 45 of the first body portion 23a such that the skin interface electrode 27 is prevented from being detached from the body 23. The skin interface electrode 27 is in the form of a grid or a mesh of conductive material. The shape of the grid or mesh of the skin interface electrode 27 can be adapted to control the flow of non-thermal plasma applied to the skin. A layer of dielectric material 28 is disposed along the main electrode 26 between the main electrode 26 and the skin interface electrode 27. The layer of dielectric material 28 is for example made of PTFE, polyoxymethylene, aluminium oxide or quartz. The layer of dielectric material 28 and the skin interface electrode 27 are arranged relative to each other in such a way that an air gap 29 locates between the layer of dielectric material 28 and the skin interface electrode 27.

The transformer 25 is configured to receive the low voltage electrical signal generated by the driving device 18, and is configured to transform the low voltage electrical signal received into a higher voltage electrical signal. Depending on the winding ratio of the transformer 25, the voltage of the low voltage electrical signal ranges between about 15V and about 30V and the voltage of the higher voltage electrical signal ranges between about 6kV and about 7kV. The voltage of the low voltage electrical signal is in particular approximately equal to 24V, and the voltage of the higher electrical signal is in particular approximately equal to 7 kV. The transformer 25 comprises a primary winding coil 30 and a secondary winding coil 31. The primary winding coil 30 is connected to the output of the driving device 18. The secondary winding coil 31 is connected to the main electrode 26 and the skin interface electrode 27 is maintained at a low or zero electrical potential, so that the higher voltage electrical signal is applied between the main electrode 26 and the skin interface electrode 27. As the skin interface electrode 27 is maintained at a low or zero electrical potential, little or no current will pass should the user accidentally touch the skin interface electrode, thereby avoiding any injury.

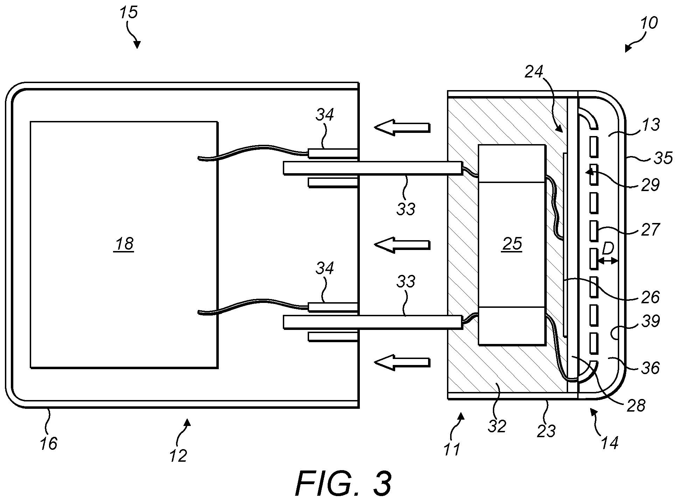

As visible in Figure 3, the transformer 25 and the main electrode 26 are embedded within a potting material 32 in the body 23. The potting material 32 is an electrically insulating material configured to electrically insulate the transformer 25 and the main electrode 26. The potting material 32 is hardenable and suitable for curing around the transformer 25 and the main electrode 26. For example, the potting material 32 is a thermosetting polymer, such as polyurethane, silicone, or epoxy resin. The potting material 32 provides electrical insulation from high electrical voltages generated within the electrode head assembly 11 during use, thereby enabling the electrode head assembly 11 to be at the same time compact and safe for the user.

The electrode head assembly 11 and the driving device 18 include cooperating elements to releasably mount the electrode head assembly 11 to the driving device 18 and electrically connect the driving device 18 to the transformer 25. The cooperating elements are in the form of a pair of plugs 33 and a pair of corresponding sockets 34. The plugs 33 are part of the electrode head assembly 11 and are electrically connected to the primary winding coil 30 of the transformer 25. The sockets 34 are arranged in the driving device 18 and electrically connected to the driving device 18. Each plug 33 is configured to respectively engage with one of the sockets 34 to releasably mount the electrode head assembly 11 to the driving device 18. When engaged with each other, the pairs of plugs 33 and sockets 34 electrically connect the driving device 18 to the primary winding coil 30 so that the low voltage electrical signal generated by the driving device 18 is transmitted to the primary winding coil 30. The device 10 is therefore configured such that no high voltage electrical signal is transmitted through the plugs 33, i.e. through the interface between the driving device 18 and the electrode head assembly 11. A user can therefore detach the electrode head assembly 11 from the driving device 18 without any risks.

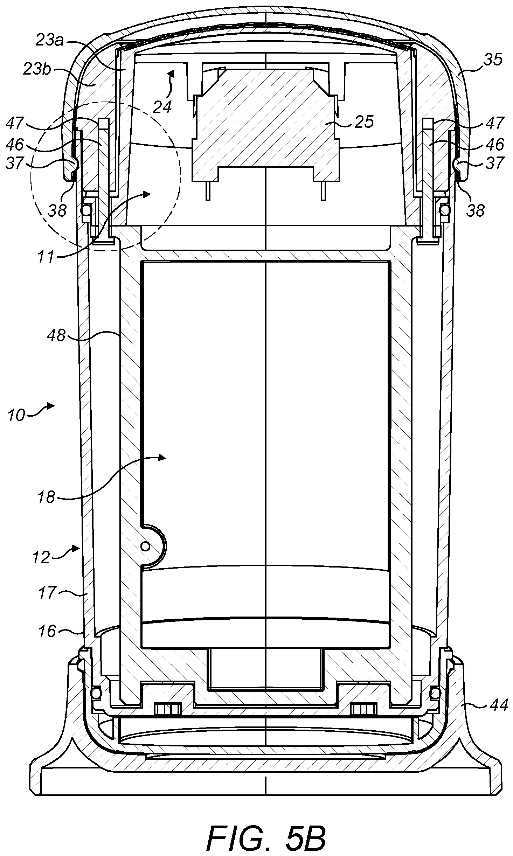

The device 10 comprises additional securing elements in the form of a pair of screws 46 and a pair of corresponding holes 47. As shown in Figures 1 and 5B, the screws 46 are attached to a sidewall 48 of the driving device 18 and the holes 47 are arranged in the body 23. Each screw 46 is configured to respectively engage with one of the holes 47 to additionally secure the electrode head assembly 11 to the driving device 18.

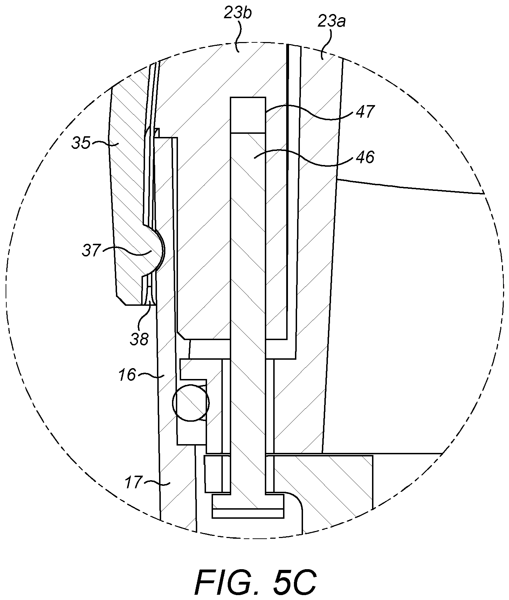

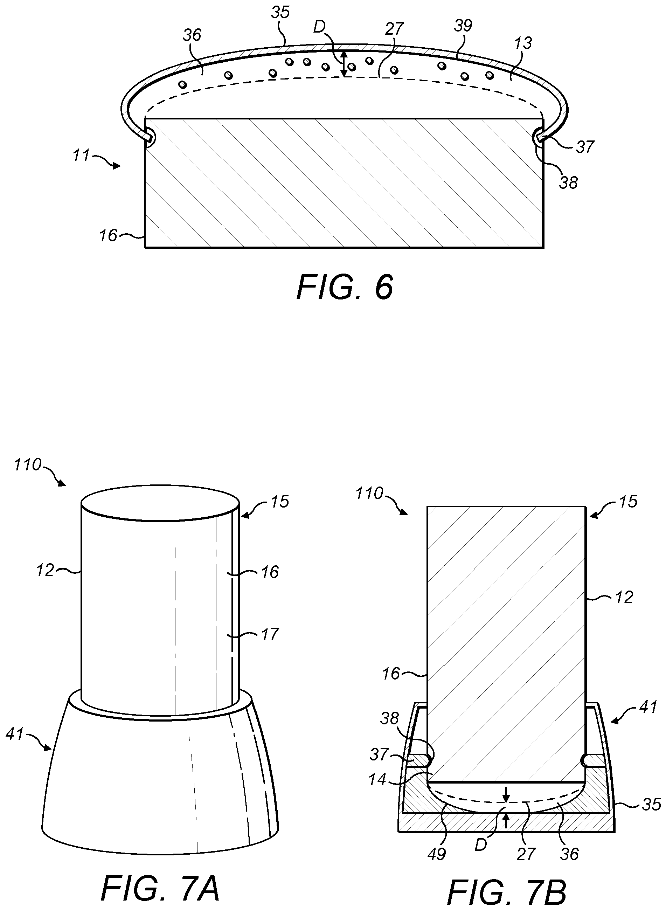

As visible in Figure 6, an isolating element 35 is provided at the skin interface 13 of the device 10. In the present embodiment, the isolating element 35 is in the form of a cap 35. The cap 35 is configured to cooperate with the housing 16. As visible in Figure 5B and 5C, the cap 35 comprises a pair of projections 37 which are configured to engage in corresponding recesses 38 formed in the sidewall 17 of the housing 16 such that an airtight seal is formed between the cap 35 and the housing 16. The cap 35 is configured to be positioned in an isolating position, in which the cap 35 isolates a region surrounding the skin interface electrode 27 to form a closed chamber 36 around the skin interface electrode 27.

When the cap 35 is in the isolating position and when non-thermal plasma is generated at the skin interface electrode 27, non-thermal plasma is contained within the closed chamber 36 about the skin interface electrode 27. In this way, the air captured in the closed chamber 36 and surrounding the skin interface electrode 27 gets saturated in ionized free particles, and the skin interface electrode 27 thereby gets cleaned and disinfected.

The cap 35 comprises an isolating surface 39 which faces the skin interface electrode 27 when the cap 35 is in the isolating position. In the isolating position, the distance D between the isolating surface 39 and the skin interface electrode 27 is at most 3 millimetres, and preferably ranges between about 0.2 millimetre and about 1 millimetre. This allows minimizing the volume of the closed chamber 36 formed around the skin interface electrode 27. In this way, the air captured in the closed chamber 36 gets more efficiently saturated in ionized free particles and an enhanced cleaning of the skin interface electrode 27 is therefore enabled.

The cap 35 is made from a rigid material such as a thermoplastic polymer. For example, the cap 35 is made from acrylonitrile butadiene styrene or polycarbonate. It will be noted that any other kind of material suitable for protecting the skin interface electrode 27 against mechanical damage can be used.

The device 10 may additionally comprise a stand 44 configured to stabilise the device 10 when the device 10 is placed in an upright position, for example on a table. The stand 44 receives the proximal end 15 of the device 10.

The operation of the skin treating device 10 in accordance with the first embodiment of the present invention will now be described.

Initially, the transformer 25 is electrically connected to the driving device 18 and the electrode head assembly 11 is mounted to the driving device 18 via the plugs and sockets 33, 34. The electrode head assembly 11 is additionally secured to the driving device 18 by fixing the screws 46 in the holes 47. In use, the driving device 18 is switched on by a user by means of the switch, and the skin interface 13 of the device 10 is positioned against or close to an area of skin to be sterilized, for example a wound. Alternatively, or more in general, the device 10 is positioned against or close to a part of the body in which bacteria are to be removed, such as an armpit. Once the driving device 18 is switched on, the driving device 18 generates a low voltage electrical signal which is transmitted to the primary winding coil 30 of the transformer 25 via the plugs and sockets 33, 34. The transformer 25 changes the low voltage electrical signal into a higher voltage electrical signal which is applied between the main electrode 26 and the skin interface electrode 27. This creates electrical discharges which ionize air located between the main electrode 26 and the skin interface electrode 27 and generate non-thermal plasma at the skin interface electrode 27. The generated non-thermal plasma diffuses onto the skin, thereby disinfecting the skin.

During treatment of the skin, the skin interface electrode 27 is positioned close to or directly against the part of the body in which bacteria are to be removed, and may therefore get contaminated. Accordingly, after treatment, the user may want to clean the skin interface electrode 27 before reusing the device 10. To this effect, the user positions the cap 35 in the isolating position so that the cap 35 is snapped on the housing 16 and forms the closed chamber 36 around the skin interface electrode 27. Then, the user switches the driving device 18 on by means of the switch. Non-thermal plasma is generated at the skin interface electrode 27 and is contained within the closed chamber 36 about the skin interface electrode 27. Therefore, the air captured in the closed chamber 36 and surrounding the skin interface electrode 27 gets saturated with ionized free particles, and the skin interface electrode 27 thereby gets cleaned and disinfected.

Alternatively, or in addition, the user may want to change of electrode head assembly 11, of may want to wash the electrode head assembly 11. To this effect, the user detaches the cap 35 from the housing 16, and detaches the electrode head assembly 11 from the driving device 18 by removing the plugs 33 from the sockets 34 and the screws 46 from the holes 47. As no high voltage electrical signal is transmitted through the plugs and sockets 33, 34 between the driving device 18 and the electrode head assembly 11, the electrode head assembly 11 can be removed from the driving device 18 without risks for the user.

A second embodiment 110 of the skin treating device according to the present invention is shown in Figures 7A and 7B. The second embodiment corresponds closely to the first embodiment and like reference numerals have been used for like components.

As shown in Figure 7A, the isolating element is in the form of a wall 49 integrally formed as part of a dock 41 to receive and support the device 110. The dock 41 is configured to receive the distal end 14 of the device 110. When the device 110 is placed in the dock 41, the wall 49 cooperates with the housing 16 to form an airtight seal between the dock 41 and the housing 16. As shown in Figure 7B, the wall 49 comprises a projection 37 which is configured to engage in a corresponding recess 38 formed in the housing 16 to form an airtight seal between the housing 16 and the dock 41 and to form the closed chamber 36 around the skin interface electrode 27.

It will be appreciated that the operation of the skin treating device 110 according to the second embodiment is unchanged from that of the first embodiment. However, in the second embodiment, when the user wants to sterilise the skin interface electrode 27, the user positions the distal end 14 of the device 110 in the dock 41 so that the projection 37 of the wall 49 engages the recess 38 in the housing 16 to form the closed chamber 36 around the skin interface electrode 27. The driving device 18 is then switched on and non-thermal plasma is generated at the skin interface electrode 27 within the closed chamber 36 so that the skin interface electrode 27 gets sterilised.

While embodiments of the invention have been illustrated and described in detail in the drawings and foregoing description, such illustration and description are to be considered illustrative or exemplary and not restrictive, and the invention not limited to these embodiments.

It will be appreciated that the term "comprising" does not exclude other elements or steps and that the indefinite article "a" or "an" does not exclude a plurality. A single processor may fulfil the functions of several items recited in the claims. The mere fact that certain measures are recited in mutually different dependent claims does not indicate that a combination of these measures cannot be used to an advantage. Any reference signs in the claims should not be construed as limiting the scope of the claims.

## Claims

1. A device (10, 110) for treating skin using non-thermal plasma comprising:
an electrode head assembly (11) having a skin interface electrode (27) for application to skin during treatment, a transformer (25) configured to change a low voltage electrical signal into a higher voltage electrical signal, and a generator (24) to receive said higher voltage electrical signal and generate non-thermal plasma at said skin interface electrode, and
a handle portion (12) containing a driving device (18) comprising a power source (19) to generate said low voltage electrical signal,
**characterised in that** the electrode head assembly and the driving device include cooperating elements (33, 34) to releasably mount the electrode head assembly to the driving device and electrically connect the power source to the transformer.

2. A device (10, 110) according to claim 1, wherein the cooperating elements (33, 34) comprise a plug (33) and a socket (34), the plug being configured to releasably engage with the socket to releasably mount the electrode head assembly to the driving device and electrically connect the power source to the transformer.

3. A device (10, 110) according to claim 2, wherein the plug (33) is part of the electrode head assembly and the socket (34) is part of the driving device.

4. A device (10, 110) according to claim 2 or claim 3, wherein the cooperating elements comprise a pair of plugs and a pair of sockets, each plug being configured to respectively releasably engage with a socket to releasably mount the electrode head assembly to the driving device and electrically connect the power source to the transformer.

5. A device (10, 110) according to any preceding claim, wherein the electrode head assembly (11) comprises a body (23), the transformer being embedded within an insulating material (32) in the body.

6. A device (10, 110) according to claim 5, wherein the insulating material (32) is hardenable and suitable for curing around the transformer.

7. A device (10, 110) according to claim 6, wherein the insulating material (32) is a thermosetting polymer.

8. A device (10, 110) according to any preceding claim, wherein the generator comprises a main electrode (26) and a dielectric material (28) disposed between the main electrode and the skin interface electrode, and wherein the main electrode is connected to the transformer so that the higher voltage electrical signal is applied to the main electrode.

9. An electrode head assembly (11) mountable to a driving device (18) comprising a power source (19), the electrode head assembly comprising a skin interface electrode (27), a transformer (25) configured to change a low voltage electrical signal received from the power source into a higher voltage electrical signal, and a generator (24) to receive said higher voltage electrical signal and generate non-thermal plasma at said skin interface electrode, **characterised in that** the electrode head assembly includes a cooperating element (33) to releasably mount the electrode head assembly to the driving device and to electrically connect the power source to the transformer.

10. An electrode head assembly (11) according to claim 9, wherein the cooperating element comprise a plug (33) configured to releasably engage with a socket (34) in the driving device to releasably mount the electrode head assembly to the driving device and electrically connect the power source to the transformer.

11. An electrode head assembly (11) according to claim 10, wherein the cooperating element comprises a pair of plugs, each plug being configured to respectively releasably engage with a socket in the driving device to releasably mount the electrode head assembly to the driving device and electrically connect the power source to the transformer.

12. An electrode head assembly (11) according to any of claims 9 to 11 comprising a body (23), wherein the transformer is located in the body and embedded within an insulating material (32) in the body.

13. An electrode head assembly (11) according to claim 12, wherein the insulating material (32) is hardenable and suitable for curing around the transformer.

14. An electrode head assembly (11) according to claim 13, wherein the insulating material (32) is a thermosetting polymer.

15. An electrode head assembly (11) according to any of claim 9 to 14, wherein the generator (24) comprises a main electrode (26) and a dielectric material (28) disposed between the main electrode and the skin interface electrode, and wherein the main electrode is connected to the transformer so that the higher voltage electrical signal is applied to the main electrode.

## Patentansprüche

1. Vorrichtung (10, 110) zur Behandlung von Haut unter Verwendung von nicht-thermischem Plasma, umfassend:
eine Elektrodenkopfbaugruppe (11) mit einer Hautoberflächenelektrode (27) zur Anwendung auf der Haut während der Behandlung, einem Transformator (25), der konfiguriert ist, um ein elektrisches Niederspannungssignal in ein elektrisches Signal höherer Spannung zu ändern, und einem Generator (24) zum Empfangen des genannten elektrischen Signals höherer Spannung und zum Erzeugen von nicht-thermischem Plasma an der genannten Hautoberflächenelektrode, und
ein Griffteil (12) enthaltend eine Antriebsvorrichtung (18) umfassend eine Stromquelle (19) zum Erzeugen des genannten elektrischen Niederspannungssignals, **dadurch gekennzeichnet, dass** die Elektrodenkopfbaugruppe und die Antriebsvorrichtung zusammenwirkende Elemente (33, 34) einschließen, um die Elektrodenkopfbaugruppe lösbar an der Antriebsvorrichtung zu montieren und die Stromquelle elektrisch mit dem Transformator zu verbinden.

2. Vorrichtung (10, 110) nach Anspruch 1, wobei die zusammenwirkenden Elemente (33, 34) einen Stecker (33) und eine Buchse (34) umfassen, wobei der Stecker konfiguriert ist, um lösbar mit der Buchse in Eingriff gebracht zu werden, um die Elektrodenkopfbaugruppe lösbar an der Antriebsvorrichtung zu montieren und die Stromquelle elektrisch mit dem Transformator zu verbinden.

3. Vorrichtung (10, 110) nach Anspruch 2, wobei der Stecker (33) Teil der Elektrodenkopfbaugruppe ist und die Buchse (34) Teil der Antriebsvorrichtung ist.

4. Vorrichtung (10, 110) nach Anspruch 2 oder Anspruch 3, wobei die zusammenwirkenden Elemente ein Steckerpaar und ein Buchsenpaar umfassen, wobei jeder Stecker konfiguriert ist, um jeweils lösbar mit einer Buchse in Eingriff gebracht zu werden, um die Elektrodenkopfbaugruppe lösbar an der Antriebsvorrichtung zu montieren und die Stromquelle elektrisch mit dem Transformator zu verbinden.

5. Vorrichtung (10, 110) nach einem der vorhergehenden Ansprüche, wobei die Elektrodenkopfbaugruppe (11) einen Körper (23) umfasst, wobei der Transformator in ein isolierendes Material (32) im Körper eingebettet ist.

6. Vorrichtung (10, 110) nach Anspruch 5, wobei das isolierende Material (32) härtbar ist und für die Aushärtung rund um den Transformator geeignet ist.

7. Vorrichtung (10, 110) nach Anspruch 6, wobei das isolierende Material (32) ein duroplastisches Polymer ist.

8. Vorrichtung (10, 110) nach einem der vorhergehenden Ansprüche, wobei der Generator eine Hauptelektrode (26) und ein dielektrisches Material (28), das zwischen der Hauptelektrode und der Hautoberflächenelektrode angeordnet ist, umfasst, und wobei die Hauptelektrode mit dem Transformator verbunden ist, so dass das elektrische Signal höherer Spannung an die Hauptelektrode angelegt wird.

9. Elektrodenkopfbaugruppe (11), montierbar an einer Antriebsvorrichtung (18) umfassend eine Stromquelle (19), wobei die Elektrodenkopfbaugruppe eine Hautoberflächenelektrode (27), einen Transformator (25), der konfiguriert ist, um ein von der Stromquelle empfangenes elektrisches Niederspannungssignal in ein elektrisches Signal höherer Spannung zu ändern, und einen Generator (24) zum Empfangen des genannten elektrischen Signals höherer Spannung und zum Erzeugen von nicht-thermischem Plasma an der genannten Hautoberflächenelektrode umfasst,
**dadurch gekennzeichnet, dass**
die Elektrodenkopfbaugruppe ein zusammenwirkendes Element (33) einschließt, um die Elektrodenkopfbaugruppe lösbar an der Antriebsvorrichtung zu montieren und die Stromquelle elektrisch mit dem Transformator zu verbinden.

10. Elektrodenkopfbaugruppe (11) nach Anspruch 9, wobei das zusammenwirkende Element einen Stecker (33) umfasst, der konfiguriert ist, um lösbar mit einer Buchse (34) in der Antriebsvorrichtung in Eingriff gebracht zu werden, um die Elektrodenkopfbaugruppe lösbar an der Antriebsvorrichtung zu montieren und die Stromquelle elektrisch mit dem Transformator zu verbinden.

11. Elektrodenkopfbaugruppe (11) nach Anspruch 10, wobei das zusammenwirkende Element ein Steckerpaar umfasst, wobei jeder Stecker konfiguriert ist, um jeweils lösbar mit einer Buchse in der Antriebsvorrichtung in Eingriff gebracht zu werden, um die Elektrodenkopfbaugruppe lösbar an der Antriebsvorrichtung zu montieren und die Stromquelle elektrisch mit dem Transformator zu verbinden.

12. Elektrodenkopfbaugruppe (11) nach einem der Ansprüche 9 bis 11, umfassend einen Körper (23), wobei sich der Transformator in dem Körper befindet und in ein isolierendes Material (32) im Körper eingebettet ist.

13. Elektrodenkopfbaugruppe (11) nach Anspruch 12, wobei das isolierende Material (32) härtbar ist und für die Aushärtung rund um den Transformator geeignet ist.

14. Elektrodenkopfbaugruppe (11) nach Anspruch 13, wobei das isolierende Material (32) ein duroplastisches Polymer ist.

15. Elektrodenkopfbaugruppe (11) nach einem der Ansprüche 9 bis 14, wobei der Generator (24) eine Hauptelektrode (26) und ein dielektrisches Material (28), das zwischen der Hauptelektrode und der Hautoberflächenelektrode angeordnet ist, umfasst, und wobei die Hauptelektrode mit dem Transformator verbunden ist, so dass das elektrische Signal höherer Spannung an die Hauptelektrode angelegt wird.

## Revendications

1. Dispositif (10, 110) de traitement de la peau au moyen d'un plasma non thermique comprenant :
un ensemble de tête d'électrode (11) ayant une électrode d'interface avec la peau (27) pour une application sur la peau pendant un traitement, un transformateur (25) configuré pour transformer un signal électrique de faible tension en un signal électrique de tension plus élevée, et un générateur (24) pour recevoir ledit signal électrique de tension plus élevée et pour générer un plasma non thermique au niveau de ladite électrode d'interface avec la peau, et
une partie de manche (12) contenant un dispositif d'entraînement (18) comprenant une source d'énergie (19) pour générer ledit signal électrique de faible tension,
**caractérisé en ce que** l'ensemble de tête d'électrode et le dispositif d'entraînement incluent des éléments de coopération (33, 34) pour monter de façon amovible l'ensemble de tête d'électrode au dispositif d'entraînement et pour connecter électriquement la source d'énergie au transformateur.

2. Dispositif (10, 110) selon la revendication 1, dans lequel les éléments de coopération (33, 34) comprennent une fiche (33) et une prise (34), la fiche étant configurée pour s'engager de façon amovible dans la prise pour monter de façon amovible l'ensemble de tête d'électrode sur le dispositif d'entrainement et pour connecter électriquement la source d'énergie au transformateur.

3. Dispositif (10, 110) selon la revendication 2, dans lequel la fiche (33) fait partie de l'ensemble de tête d'électrode et la prise (34) fait partie du dispositif d'entraînement.

4. Dispositif (10, 110) selon la revendication 2 ou la revendication 3, dans lequel les éléments de coopération comprennent une paire de fiches et une paire de prises, chaque fiche étant configurée pour s'engager respectivement de façon amovible dans une prise pour monter de façon amovible l'ensemble de tête d'électrode sur le dispositif d'entrainement et pour connecter électriquement la source d'énergie au transformateur.

5. Dispositif (10, 110) selon une quelconque revendication précédente, dans lequel l'ensemble de tête d'électrode (11) comprend un corps (23), le transformateur étant noyé à l'intérieur d'un matériau isolant (32) dans le corps.

6. Dispositif (10, 110) selon la revendication 5, dans lequel le matériau isolant (32) est durcissable et adapté à un durcissement autour du transformateur.

7. Dispositif (10, 110) selon la revendication 6, dans lequel le matériau isolant (32) est un polymère thermodurcissable.

8. Dispositif (10, 110) selon une quelconque revendication précédente, dans lequel le générateur comprend une électrode principale (26) et un matériau diélectrique (28) disposé entre l'électrode principale et l'électrode d'interface avec la peau, et dans lequel l'électrode principale est connectée au transformateur de sorte que le signal électrique de tension plus élevée soit appliqué à l'électrode principale.

9. Ensemble de tête d'électrode (11) pouvant être monté sur un dispositif d'entraînement (18) comprenant une source d'énergie (19), l'ensemble de tête d'électrode comprenant une électrode d'interface avec la peau (27), un transformateur (25) configuré pour transformer un signal électrique de faible tension reçu de la source d'énergie en un signal électrique de tension plus élevée, et un générateur (24) pour recevoir ledit signal électrique de tension plus élevée et pour générer un plasma non thermique au niveau de ladite électrode d'interface avec la peau,
**caractérisé en ce que** l'ensemble de tête d'électrode inclut un élément de coopération (33) pour monter de façon amovible l'ensemble de tête d'électrode au dispositif d'entraînement et pour connecter électriquement la source d'énergie au transformateur.

10. Ensemble de tête d'électrode (11) selon la revendication 9, dans lequel l'élément de coopération comprend une fiche (33) configurée pour s'engager de façon amovible dans une prise (34) dans le dispositif d'entraînement pour monter de façon amovible l'ensemble de tête d'électrode sur le dispositif d'entrainement et pour connecter électriquement la source d'énergie au transformateur.

11. Ensemble de tête d'électrode (11) selon la revendication 10, dans lequel l'élément de coopération comprend une paire de fiches, chaque fiche étant configurée pour s'engager respectivement de façon amovible dans une prise dans le dispositif d'entraînement pour monter de façon amovible l'ensemble de tête d'électrode sur le dispositif d'entrainement et pour connecter électriquement la source d'énergie au transformateur.

12. Ensemble de tête d'électrode (11) selon l'une quelconque des revendications 9 à 11, comprenant un corps (23), dans lequel le transformateur est situé dans le corps et est noyé à l'intérieur d'un matériau isolant (32) dans le corps.

13. Ensemble de tête d'électrode (11) selon la revendication 12, dans lequel le matériau isolant (32) est durcissable et adapté à un durcissement autour du transformateur.

14. Ensemble de tête d'électrode (11) selon la revendication 13, dans lequel le matériau isolant (32) est un polymère thermodurcissable.

15. Ensemble de tête d'électrode (11) selon l'une quelconque des revendications 9 à 14, dans lequel le générateur (24) comprend une électrode principale (26) et un matériau diélectrique (28) disposé entre l'électrode principale et l'électrode d'interface avec la peau, et dans lequel l'électrode principale est connectée au transformateur de sorte que le signal électrique de tension plus élevée soit appliqué à l'électrode principale.
